# EUROPEAN PATENT APPLICATION

(11) **EP 4 803 056 A1**
(43) Date of publication of application: **09.09.2026**
(21) Application number: 24885685.8
(22) Date of filing: 29.10.2024
(51) Int. Cl.: A61F 9/007, A61K 35/30, A61P 27/02, C12M 3/00, C12N 5/071

(54) **TRANSPLANTATION DEVICE CAPABLE OF ENCAPSULATING CELLS THEREIN AND USE THEREOF**

(30) Priority: 30.10.2023 JP 2023185161
(71) Applicant: VCCT Inc., Kobe-shi, Hyogo 650-0047 (JP); The University of Tokyo, Bunkyo-ku, Tokyo 113-8654 (JP)
(72) Inventor: TAKAHASHI Masayo, Kobe-shi, Hyogo 650-0047 (JP); FUTATSUGI Yoko, Kobe-shi, Hyogo 650-0047 (JP); TAKEUCHI Shoji, Tokyo 113-8654 (JP); NIE Minghao, Tokyo 113-8654 (JP)
(74) Representative: Henderson, Helen Lee
(86) International application number: PCT/JP2024/038435
(87) International publication number: WO 2025/094914

(57) **Abstract**

The present invention relates to a transplantation device capable of encapsulating cells, a cell-based pharmaceutical product including the transplantation device preloaded with a transplantation material containing cells, a method for producing the cell-based pharmaceutical product, and the like. More specifically, the present invention relates to a transplantation device that includes an inner tube and an outer tube, in which the inner tube is a tubular structure that includes a microtube that is capable of encapsulating a transplantation material containing cells and composed of a semipermeable membrane and an injection portion provided with an injection port for supplying the cells to the microtube, the outer tube is an injection needle or cannula that includes a needle tube portion having an inner diameter capable of accommodating the microtube, and the transplantation device is used by attaching the inner tube to the outer tube. The present invention relates to a cell-based pharmaceutical product including the transplantation device preloaded with the transplantation material containing cells, a method for producing the cell-based pharmaceutical product, and the like.

## Description

### CROSS-REFERENCE TO RELATED APPLICATION:

This application claims priority from Japanese Patent Application No. 2023-185161 filed with the Japan Patent Office on October 30, 2023, the entire content of which is hereby incorporated by reference.

### TECHNICAL FIELD:

The present invention relates to a transplantation device capable of encapsulating cells, a cell-based pharmaceutical product comprising the transplantation device preloaded with a transplantation material containing cells, a method for producing the cell-based pharmaceutical product, and the like.

### BACKGROUND ART

In regeneration medicine, it is necessary to culture and expand cells ex vivo and to prepare a sufficient quantity of high-quality cells. As a technique for culturing and expanding cells ex vivo, a method is known in which cells are cultured and proliferated using a lumen of a hollow fiber composed of a semipermeable membrane (Patent Document 1). It has been reported that even cells that are difficult to proliferate, such as ES cells and iPS cells, can be cultured in large quantities by using hollow fibers (Non-patent Documents 1, 2, and Patent Document 2). However, in conventional reports, methods for supplying cells expanded within hollow fibers to medical settings in a state suitable for transplantation have not been studied.

For cell transplantation, methods such as applying cells in the form of a cell suspension, applying cells as a cell sheet, and applying cells as organoids are used. While the cell suspension is easy to prepare and can be transplanted with small surgical invasiveness, it is difficult to control the positioning of cells at the transplantation site, and it is not possible to cover a wide area. On the other hand, while the cell sheet can cover a wide area of the transplantation site, their preparation requires time and necessitates invasive surgical procedures involving large incisions. The production of organoids requires advanced techniques and time.

The present inventors have reported that, in transplantation of retinal pigment epithelial (RPE) cells, by forming the cells into a string-like aggregate, the cells can be easily injected into the subretinal space and can cover a certain area similarly to a sheet (Patent Document 3). For the transplantation of the string-like aggregate, it is necessary to collect the produced aggregate from the device, place it in a dish, transport it to a transplantation facility, and load it into a transplantation instrument. Accordingly, the transport range of the cells is limited to within walking distance, and loading into a transplantation needle requires the skill of a trained operator. Therefore, for practical application, there has been a need for the development of dosage forms and techniques that facilitate transport and loading into transplantation instruments.

### CITED DOCUMENTS

### PATENT LITERATURE

Patent Document 1: JP-A-2016-07207
Patent Document 2: JP-A-2018-050498
Patent Document 3: WO2022/230977

### NON-PATENT LITERATURE

Non-patent Document 1: Fujii et al. Cytotechnology. 2020 Apr; 72(2): 227-237
Non-patent Document 2: Matsushita et al. J Biosci Bioeng. 2019 Oct; 128(4): 480-486

### DISCLOSURE OF THE INVENTION

### PROBLEMS TO BE SOLVED BY THE INVENTION

It is an object of the present invention to provide means for supplying high-quality cells to medical settings in a state suitable for transplantation, provide a cell preparation that can be used without requiring specialized skills of a trained operator, or the like.

### SOLUTIONS TO THE PROBLEMS

The present inventors have found that, by culturing cells in a lumen of a microtube (hollow fiber) composed of a semipermeable membrane, the cells can be proliferated and obtained as aggregates, and that the proliferated cells can be transported together with a culture container in a state in which the cells are contained within the microtube, and can be easily mounted onto a transplantation needle and used in a medical setting.

The present invention is based on the above-described knowledge, and provides [1] to [18] described below.
[1] A transplantation device comprising:
   an inner tube; and
   an outer tube, wherein
   the inner tube is a tubular structure that includes a microtube that is capable of encapsulating a transplantation material containing cells and composed of a semipermeable membrane and an injection portion provided with an injection port for supplying the cells to the microtube,
   the outer tube is an injection needle or cannula that includes a needle tube portion having an inner diameter capable of accommodating the microtube, and
   the transplantation device is used by attaching the inner tube to the outer tube.
[2] A transplantation device as a tubular structure comprising
   a microtube that is capable of encapsulating a transplantation material containing cells and composed of a semipermeable membrane; and
   an injection portion provided with an injection port for supplying the cells to the microtube, wherein
   the transplantation device has a structure configured to be used by being attached to an interior of an injection needle or cannula that includes a needle tube portion having an inner diameter capable of accommodating the microtube.
[3] The transplantation device according to [1] or [2], wherein
   the semipermeable membrane is composed of one or a combination of two or more selected from the group consisting of polylactic acid (PLA), polyglycolic acid (PGA), lactic acid-glycolic acid copolymer (PLGA), polyhydroxy acids, polycaprolactone, polycarbonate, polyamide, polyethylene, polyurethane, polyarylate, polysulfone, polyethersulfone, polyester, polystyrene, polyvinyl alcohol, polyvinyl acetate, polyvinyl chloride, polyvinyl fluoride, polyvinyl imidazole, chlorosulfonated polyolefin, polyethylene oxide, polyphosphazene, polyamino acids, polyorthoester, polyacetal, polycyanoacrylate, polytetrafluoroethylene (PTFE), biodegradable polyurethane, polyvinylidene fluoride, polytetrafluoroethylene, cellulose acetate, polyacrylonitrile, polyacrylate, ethylene-vinyl acetate polymer, acyl-substituted cellulose acetate, polymethyl methacrylate, polypropylene, regenerated cellulose, and derivatives thereof.
[4] The transplantation device according to any of [1] to [3], wherein
   the microtube has an inner diameter of 20 µm to 2000 µm.
[5] The transplantation device according to any of [1] to [4], wherein
   the semipermeable membrane has a pore diameter of 0.01 µm to 10 µm.
[6] The transplantation device according to any of [1] to [5] further comprising one or more of (1) to (3) below:
   (1) an end portion of the microtube at an opposite side of the injection port is sealable;
   (2) the injection port is sealable; and
   (3) the injection needle or cannula includes an attachment portion to an external surgical device.
[7] A cell-based pharmaceutical product comprising
   a transplantation device preloaded with a transplantation material containing cells, wherein
   the transplantation device includes an inner tube and an outer tube,
   the inner tube is a tubular structure that includes a microtube that is capable of encapsulating the transplantation material containing cells and composed of a semipermeable membrane and an injection portion provided with an injection port for supplying the cells to the microtube,
   the outer tube is an injection needle or cannula that includes a needle tube portion having an inner diameter capable of accommodating the microtube, and
   the cell-based pharmaceutical product is used with the inner tube attached to the outer tube.
[8] A cell-based pharmaceutical product comprising
   a transplantation device preloaded with a transplantation material containing cells, wherein
   the transplantation device is a tubular structure including a microtube that is capable of encapsulating a transplantation material containing cells and composed of a semipermeable membrane and an injection portion provided with an injection port for supplying the cells to the microtube, and
   the transplantation device has a structure configured to be used by being attached to an interior of an injection needle or cannula that includes a needle tube portion having an inner diameter capable of accommodating the microtube.
[9] The cell-based pharmaceutical product according to [7] or [8], wherein
   the cells are one or two or more selected from stem cells, progenitor cells, somatic cells, and cells induced to differentiate from the stem cells or the progenitor cells.
[10] The cell-based pharmaceutical product according to any of [7] to [9], wherein
   the cells include retinal pigment epithelial cells.
[11] The cell-based pharmaceutical product according to any of [7] to [10], wherein
   at least a part of the cells forms an aggregate in the microtube.
[12] The cell-based pharmaceutical product according to any of [7] to [11], wherein
   the semipermeable membrane is composed of one or a combination of two or more selected from the group consisting of polylactic acid (PLA), polyglycolic acid (PGA), lactic acid-glycolic acid copolymer (PLGA), polyhydroxy acids, polycaprolactone, polycarbonate, polyamide, polyethylene, polyurethane, polyarylate, polysulfone, polyethersulfone, polyester, polystyrene, polyvinyl alcohol, polyvinyl acetate, polyvinyl chloride, polyvinyl fluoride, polyvinyl imidazole, chlorosulfonated polyolefin, polyethylene oxide, polyphosphazene, polyamino acids, polyorthoester, polyacetal, polycyanoacrylate, polytetrafluoroethylene (PTFE), biodegradable polyurethane, polyvinylidene fluoride, polytetrafluoroethylene, cellulose acetate, polyacrylonitrile, polyacrylate, ethylene-vinyl acetate polymer, acyl-substituted cellulose acetate, polymethyl methacrylate, polypropylene, regenerated cellulose, and derivatives thereof.
[13] The cell-based pharmaceutical product according to any of [7] to [12], wherein
   the microtube has an inner diameter of 20 µm to 2000 µm.
[14] A method for producing the cell-based pharmaceutical product according to any of [7] to [13], comprising:
   preparing the transplantation device; and
   injecting a culture medium containing cells into the microtube of the tubular structure of the transplantation device and culturing the cells by placing the tubular structure in a reservoir of a culture container filled with a culture medium.
[15] The method according to [14], comprising
   sealing, after the culturing, an end portion of the microtube at an opposite side of the injection port.
[16] The method according to [14] or [15], comprising
   sealing the injection port of the tubular structure after the culturing, and holding the tubular structure in the reservoir under anoxic conditions.
[17] A kit comprising:
   the transplantation device according to any one of [1] to [6]; and
   a culture container, wherein
   the culture container includes a reservoir configured to be filled with a culture medium and to allow cell culture with the tubular structure placed in the reservoir.
[18] The kit according to [17], wherein
   the culture container is capable of holding the tubular structure in the reservoir under anoxic conditions.

The transplantation device according to [1] is a combination of the transplantation device according to [2] that serves as the inner tube and an injection needle or cannula that serves as the outer tube, and the cell-based pharmaceutical product according to [7] is a combination of the cell-based pharmaceutical product according to [8] and an injection needle or cannula that serves as the outer tube of the transplantation device.

Accordingly, [2], [7], and [8] can be also described as follows.
[2'] A transplantation device that constitutes the inner tube of the transplantation device according to [1], the transplantation device being a tubular structure comprising a microtube that is capable of encapsulating a transplantation material containing cells and composed of a semipermeable membrane and an injection portion provided with an injection port for supplying the cells to the microtube, wherein
   the transplantation device has a structure configured to be used by being attached to an interior of an injection needle or cannula that includes a needle tube portion having an inner diameter capable of accommodating the microtube.
[7'] A cell-based pharmaceutical product in which a transplantation material containing cells is preloaded in the inner tube of the transplantation device according to [1].
[8'] A cell-based pharmaceutical product in which a transplantation material containing cells is preloaded in the inner tube of the transplantation device according to [2].

### EFFECTS OF THE INVENTION

According to the present invention, cells can be proliferated and expanded in a lumen of a microtube (hollow fiber) composed of a semipermeable membrane, and obtained as aggregates. Since the proliferated cells can be transported in a state of being contained in the microtube together with the culture container and thus the quality thereof can be maintained for a certain period, the transportable range of the cells is expanded compared with conventional methods. Furthermore, since the microtube can be easily attached to the transplantation needle for use, it does not require the skilled technique of aspirating cell aggregates from a dish or the like and loading them into a transplantation needle.

### BRIEF DESCRIPTION OF THE DRAWINGS

Fig. 1 is a diagram illustrating an exemplary inner tube (tubular structure) of a transplantation device of the present invention.
Fig. 2 is a diagram illustrating an exemplary outer tube (injection needle or cannula) of the transplantation device of the present invention.
Fig. 3 is a diagram schematically illustrating culturing and transport methods of the transplantation device of the present invention, Fig. 3A illustrates an aspect in which a plurality of inner tubes (tubular structures) are provided in a culture container, and Fig. 3B illustrates an aspect in which one inner tube (tubular structure) is provided in the culture container.
Fig. 4 is a diagram schematically illustrating a method for using the transplantation device of the present invention.
Fig. 5 is a microscopic image obtained when a string-like aggregate was seeded in a culture dish and cultured for 2 days, and release of individual retinal pigment epithelial cells from fragmented aggregates (arrows) tended to occur earlier than the release from robust aggregates (arrowheads).
Fig. 6 is a photomicrograph obtained when a string-like aggregate was placed in a container filled with a culture medium, sealed, stored under anoxic conditions for 13 days, then seeded in a culture dish, and cultured for 14 days, from the string-like aggregate that had undergone the storage, retinal pigment epithelial cells migrated in a manner similar to that from the string-like aggregate that had not undergone the storage, and an engraftment area of the individual retinal pigment epithelial cells continued to expand during the 14-day culture period.
Fig. 7 is a diagram schematically illustrating (a) production, (b) transport, and (c) use of a transplantation device including retinal pigment epithelial cells.
Fig. 8A is a photograph of an inner tube (tubular structure) filled with a human hepatocellular carcinoma-derived cell line, HepG2, Fig. 8B is a photomicrograph of cells extracted from the microtube after culture, and the cells after the culture form an aggregate.

### DESCRIPTION OF PREFERRED EMBODIMENTS

### 1. Transplantation Device

### 1.1 Transplantation Device I

In a first embodiment, a transplantation device of the present invention includes two members, an inner tube and an outer tube. Hereinafter the transplantation device is referred to as a "transplantation device I."

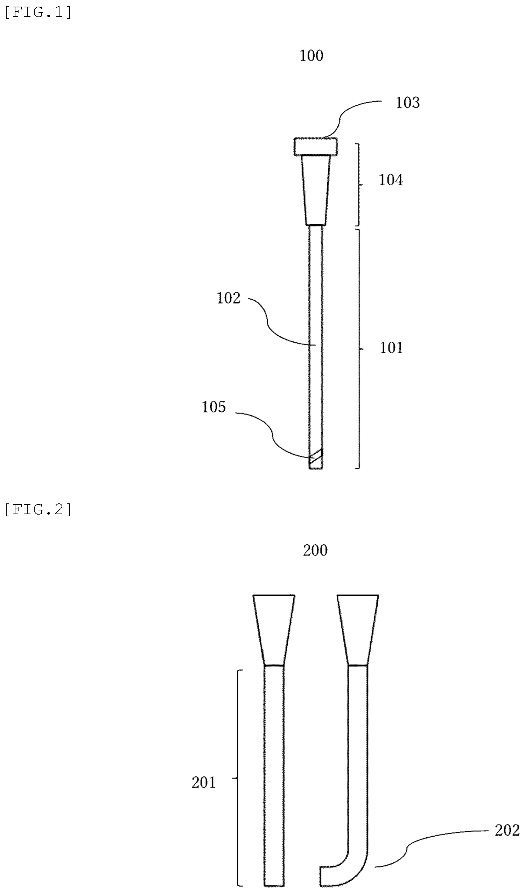

Fig. 1 illustrates an exemplary inner tube (100). The "inner tube" is a tubular structure that includes a "microtube" (101) composed of a semipermeable membrane capable of encapsulating a transplantation material containing cells and an "injection portion" (104) provided with an injection port (103) for supplying cells to the microtube. The injection port may have a sealable structure. An end portion of the microtube at the opposite side of the injection port may be configured to be sealable (105).

The "semipermeable membrane" constituting the microtube of the inner tube is a membrane that does not allow cells to pass therethrough, but allows substances necessary for culturing and proliferation of the cells to pass into the interior of the microtube and allows unnecessary waste products to pass to the outside of the microtube. Examples of materials for the semipermeable membrane include one or a combination of two or more selected from the group consisting of polylactic acid (PLA), polyglycolic acid (PGA), lactic acid-glycolic acid copolymer (PLGA), polyhydroxy acids, polycaprolactone, polycarbonate, polyamide, polyethylene, polyurethane, polyarylate, polysulfone, polyethersulfone, polyester, polystyrene, polyvinyl alcohol, polyvinyl acetate, polyvinyl chloride, polyvinyl fluoride, polyvinyl imidazole, chlorosulfonated polyolefin, polyethylene oxide, polyphosphazene, polyamino acids, polyorthoester, polyacetal, polycyanoacrylate, polytetrafluoroethylene (PTFE), biodegradable polyurethane, polyvinylidene fluoride, polytetrafluoroethylene, cellulose acetate, polyacrylonitrile, polyacrylate, ethylene-vinyl acetate polymer, acyl-substituted cellulose acetate, polymethyl methacrylate, polypropylene, regenerated cellulose, and derivatives thereof.

The semipermeable membrane preferably has a pore diameter of 0.01 µm to 5 µm. By adjusting the pore diameter of the semipermeable membrane, the permeation of substances into and out of the microtube can be controlled, and the cells can be favorably maintained.

A thickness of the semipermeable membrane is preferably from 10 µm to 100 µm, and more preferably from 10 µm to 50 µm. By adjusting the thickness of the semipermeable membrane, the permeation of substances into and out of the microtube can be controlled, and the shape retention (strength) of the microtube can also be controlled.

The microtube composed of a semipermeable membrane is, for example, a hollow fiber (made of a semipermeable membrane).

An inner diameter of the microtube (hollow fiber) is not specifically limited insofar as it is capable of encapsulating cells required for transplantation and allowing favorable proliferation thereof. For example, the inner diameter of the semipermeable membrane is from 20 µm to 2000 µm, and preferably from 150 µm to 270 µm.

Although a length of the microtube (hollow fiber) is not specifically limited insofar as it is capable of encapsulating cells required for transplantation and allowing favorable proliferation thereof, it is preferably several millimeters longer than the length of the needle tube portion of the outer tube (see 404). This is because, when the inner tube is attached to the outer tube for use, the length of the microtube can be adjusted by cutting the distal end of the microtube protruding from the needle tube portion with scissors or the like, thereby releasing the sealing at a distal end (404), and because, in the case of subretinal transplantation in ophthalmic surgery, transplantation of the encapsulated cells can be performed by insertion of only the inner tube without inserting the outer tube into the subretinal space.

The inner diameter and the length of the microtube (hollow fiber) are appropriately optimized within the above-described ranges according to the purpose of transplantation and the administration site. For example, in the case of ophthalmic surgery, assuming that the injection needle to be used has a diameter of 25G (0.5 mm) and a length of about 28 mm, the microtube constituting the inner tube has an outer diameter of about 70 µm to 290 µm (31G to 38G) and a length of about 33 mm to 39 mm so as to be mountable within the injection needle. In this case, considering the thickness of the semipermeable membrane (10 µm to 50 µm), the inner diameter of the microtube is about 50 µm to 270 µm.

At one end portion of the microtube, the "injection portion" (104) including an injection port (103) for supplying cells to the microtube is provided. The injection port may have a sealable structure, and for example, a plug, cap, or seal having water resistance and air barrier properties may be attached to the injection port (see 305). As described later, since it is sufficient that the inner tube (tubular structure) is sealed in a state of being contained in a culture container, sealing of the inner tube itself is not necessarily required.

End portions (105, 304) of the microtubes at the opposite side of the injection port may be open, or may be sealable. As described above, the sealing of the sealed end portion can be easily released by cutting the microtube protruding from the outer tube (injection needle or needle tube portion of the cannula) with scissors or the like after attaching the inner tube to the outer tube (see 404).

The "cells" encapsulated in the microtube are not particularly limited, and may be any of stem cells, progenitor cells, or somatic cells. Examples of stem cells and progenitor cells include pluripotent stem cells, such as embryonic stem cells (ES cells) and induced pluripotent stem cells (iPS cells); and tissue stem cells and progenitor cells, such as hematopoietic stem cells and progenitor cells, neural stem cells and progenitor cells, hepatic stem cells and progenitor cells, pancreatic stem cells and progenitor cells, skin stem cells and progenitor cells, osteochondral stem cells and progenitor cells, and mesenchymal stem cells including adipose-derived stem cells, cardiac stem cells, and dental pulp stem cells. Examples of somatic cells include differentiated cells such as lymphocytes, epithelial cells, endothelial cells, muscle cells, fibroblasts (skin cells and the like), hair cells, hepatocytes, gastric mucosal cells, intestinal cells, splenocytes, pancreatic cells (pancreatic exocrine cells and the like), brain cells, lung cells, renal cells, ocular-related cells, adipocytes, and neural glial cells. The differentiated cells may be those derived from pluripotent stem cells or from stem cells and progenitor cells.

As the ES cells, various established ES cell lines may be used. Examples of ES cell lines include clinical-grade human ES cell lines provided by Center for Human ES Cell Research, Institute for Life and Medical Sciences, Kyoto University, and human ES cell lines (KhES-1, KhES-1_Crx::Venus, KhES-1_Rx::Venus) provided by RIKEN BRC; however, the ES cell lines are not limited thereto.

As the iPS cells, not only iPS cells derived from somatic cells of a patient, but also various iPS cell lines may be used. Examples of iPS cell lines include various iPS stock cell lines provided by CiRA Foundation (Center for iPS Cell Research and Application, Kyoto University), healthy donor-derived iPS cell lines provided by RIKEN BRC, and KVs09 and CLs23; however, the iPS cell lines are not limited thereto.

The biological species of the cells is not particularly limited and is appropriately selected depending on the intended purpose. For human transplantation, cells derived from primates, particularly cells derived from monkeys or humans, are preferred, and cells derived from humans are more preferred.

The transplantation device of the present invention can be appropriately used particularly for cells administered to a transplantation site using an injection needle or a cannula. Examples of such cells include ocular-related cells such as corneal epithelial cells, retinal pigment epithelial cells, neural retinal cells, conjunctival epithelial cells, limbal epithelial cells, corneal endothelial cells, corneal stromal cells, iris stromal cells, scleral cells, iris pigment epithelial cells, ciliary epithelial cells, optic nerve cells, sublimbal fibroblasts, subconjunctival fibroblasts, lacrimal gland cells, meibomian gland cells, goblet cells, lens epithelial cells, and eyelid epithelial cells. Preferred ocular-related cells are retinal pigment epithelial cells, neural retinal cells, and lacrimal gland cells, and particularly preferred ocular-related cells are retinal pigment epithelial cells. In addition to ocular-related cells, pancreatic ***β*** cells, hepatic lineage cells, neural glial cells, and neural progenitor cells are also suitable for the transplantation device of the present invention.

Fig. 2 illustrates an exemplary outer tube (200). The "outer tube" is an injection needle or a cannula provided with a needle tube portion (201) having an inner diameter capable of accommodating the microtube (101). A diameter (outer diameter) of the needle tube portion of the outer tube (injection needle or cannula) is selected according to the purpose of surgery and an administration site. A distal end (202) of the injection needle or cannula may be straight or curved.

The injection needle or cannula may include an attachment portion to an external surgical device. Examples of such external surgical devices include a syringe and Constellation VFQ.

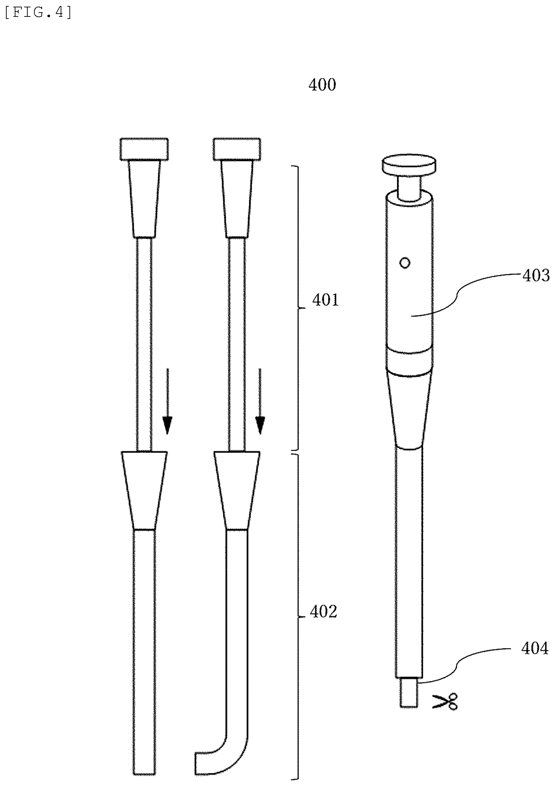

The inner tube and the outer tube are provided as a set (kit), and are used in a medical setting with the inner tube attached to the outer tube (see Fig. 4). Therefore, the inner tube (tubular structure) has a structure configured to be used by being attached inside the outer tube (injection needle or cannula).

### 1.2 Transplantation Device II

In a second embodiment, a transplantation device of the present invention is a tubular structure having the same configuration as the above-described inner tube (100), and is used by being attached inside a commercially available injection needle or cannula as an outer tube (see Fig. 1). Hereinafter the transplantation device is referred to as a "transplantation device II."

The transplantation device II is a tubular structure that includes a microtube (101) composed of a semipermeable membrane capable of encapsulating a transplantation material (102) containing cells and an injection portion (104) provided with an injection port (103) for supplying cells to the microtube, and the transplantation device II has a structure configured to be used by being attached to an interior of an injection needle or cannula (for example, MedOne0 (registered trademark), Poly Tip (registered trademark), Cannula 25g/31g or the like) that includes a needle tube portion having an inner diameter capable of accommodating the microtube.

As the structure to be used by being attached inside the injection needle or cannula, methods known in the field, for example, a structure in which the injection portion of the tubular structure comes into close contact and/or engages with a base portion of the injection needle or cannula, may be used. Additionally, the structure of the microtube constituting the transplantation device is as described in 1.1.

### 2. Cell-Based Pharmaceutical Product

The present invention provides a cell-based pharmaceutical product obtained by preloading a transplantation material containing cells in the transplantation device.

### 2.1 Cell-Based Pharmaceutical Product I

In the first embodiment, the transplantation material (102) containing cells is preloaded in the transplantation device I. As described above, the transplantation device I includes the inner tube (100) and the outer tube (200), the inner tube is a tubular structure that includes the microtube (101) composed of a semipermeable membrane capable of encapsulating the transplantation material containing cells and the injection portion (104) provided with the injection port (103) for supplying the cells to the microtube, and the outer tube is an injection needle or cannula provided with the needle tube portion (201) having an inner diameter capable of accommodating the microtube. The transplantation device I is used by attaching the inner tube to the outer tube.

### 2.2 Cell-Based Pharmaceutical Product II

In the second embodiment, the transplantation material (102) containing cells is preloaded in the microtube of the transplantation device II. As described above, the transplantation device II is a tubular structure that includes the microtube (101) composed of a semipermeable membrane capable of encapsulating the transplantation material containing cells and the injection portion (104) provided with the injection port (103) for supplying the cells to the microtube, and has the structure to be used by being attached to the interior of the injection needle or cannula provided with the needle tube portion having the inner diameter capable of accommodating the microtube.

The cells used for the cell-based pharmaceutical product of the present invention are cells described as the "'cells' encapsulated in the microtube" as defined in 1. At least a part of the cells in the microtube may constitute an aggregate. In the case of adhesive cells, cells cultured and proliferated in the microtube composed of a semipermeable membrane form aggregates. In the case of retinal pigment epithelial cells, the aggregate formed is similar to a string-like aggregate produced by a device described in WO2022/230977, while also including smaller fragments. The present inventors have confirmed that such fragments may likewise exhibit high effectiveness as a transplant material similarly to the string-like aggregate (see Test Examples described below).

The cell-based pharmaceutical product of the present invention may contain a pharmaceutically acceptable carrier or medium, specifically, sterile water, physiological saline, a culture medium, physiological buffers such as PBS, preservatives, surfactants, stabilizers, excipients, antiseptics, binders, reducing agents, and isotonic agents. If necessary, a cryoprotectant may be added, and the product may be cryopreserved and thawed for use.

### 3. Producing Method for Cell-Based Pharmaceutical Product

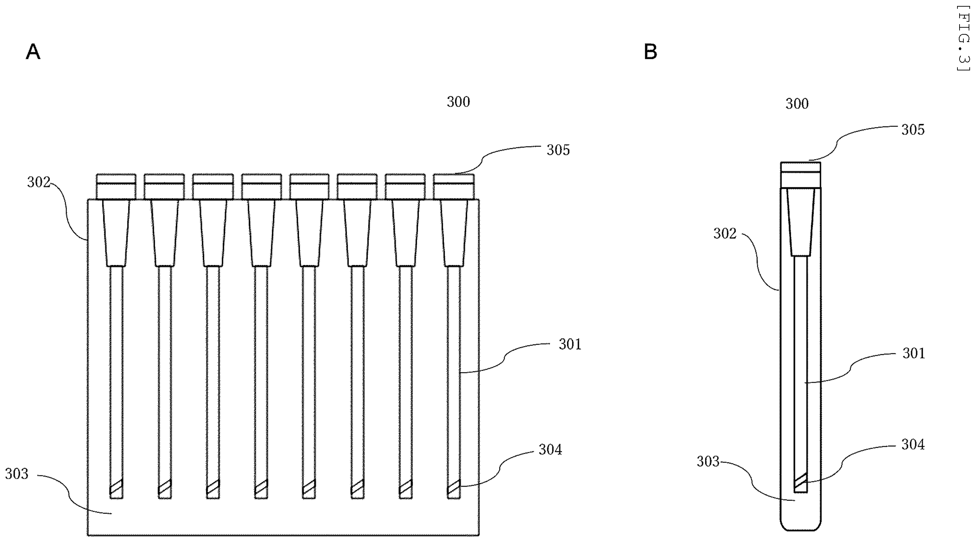

Fig. 3 schematically illustrates a culture container used in the production of the cell-based pharmaceutical product. The cell-based pharmaceutical product of the present invention can be produced by injecting a culture medium containing cells into a microtube (301) of an inner tube (tubular structure), and placing the tubular structure in a reservoir (303) of a culture container (302) filled with a culture medium and culturing the cells. The number of the inner tubes placed in the culture container is not particularly limited, and may be one or two or more. When a large number of cell-based pharmaceutical products can be stocked in a medical setting while being contained in a culture container, several of them can be used for each surgery.

The cell culture is performed by setting the culture container (302) in a culture apparatus. The culture apparatus may have known means necessary for cell culture, such as means for controlling culture temperature, means for controlling oxygen and carbon dioxide concentrations, means for exchanging the culture medium, and means for supplying additional components.

The culture medium (medium) and culture conditions are appropriately determined depending on the cells. The cell density in the microtube is 5 × 10⁷ cells/mL or more, preferably 5 × 10⁷ cells/mL to 1 × 10⁸ cells/mL, and more preferably, approximately, 7.5 × 10⁷ cells/mL to 1 × 10⁸ cells/mL.

After culturing, the end portion (304) of the microtube at the opposite side of the injection port may be sealed. As the sealing method, a seal, welding, or a hollow fiber having a closed end may be used.

The inner tube (tubular structure) is preferably sealable in a state of being contained in the culture container. This enables maintaining the quality of the loaded transplantation material. For example, by using a sealable container as the culture container, it is possible to seal the inner tube (tubular structure).

The culture container is removably attachable to the culture apparatus, and can be removed from the culture apparatus after completion of the cell culture and transported in a state where the tubular structure is arranged therein. Therefore, the cell-based pharmaceutical product can be provided to a medical institution in a state where the transplant material is loaded therein without freezing.

### 4. Transport Method for Cell-Based Pharmaceutical Product

The cell-based pharmaceutical product can be transported in a state where the tubular structure is arranged in the culture container. In the case of transport under anoxic conditions, the quality of the cells encapsulated in the tubular structure (microtube) can be maintained for about 30 days.

The present specification also discloses a kit used for production and transport of the cell-based pharmaceutical product. The kit includes the transplantation device I or the transplantation device II and the culture container (302) as indispensable components. As described above, the culture container includes the reservoir that can be loaded with the culture medium, the cell culture can be performed with the tubular structure constituting the transplantation device arranged in the reservoir, and furthermore, it is preferred that the tubular structure can be maintained in the reservoir under anoxic conditions. Additionally, details of the culture container is as described in "3. Producing Method for Cell-Based Pharmaceutical Product."

The cell-based pharmaceutical product (transplantation material) of the present invention may be frozen in a state of being loaded in the tubular structure (inner tube). In the case of freezing, the tubular structure after culture is removed from the culture container and transported in the frozen state. In this case, the cell-based pharmaceutical product I may be transported in a state where the outer tube and the inner tube are combined as a kit.

### 5. Method for Using Cell-Based Pharmaceutical Product

Fig. 4 schematically illustrates a method for using the cell-based pharmaceutical product. The cell-based pharmaceutical product of the present invention is used by attaching a tubular structure (401) as the inner tube to an injection needle or cannula (402) as the outer tube. The outer tube is appropriately mounted to an external surgical device such as a syringe (403). The length of the microtube of the tubular structure is designed to be longer than the length of the needle tube portion of the injection needle or cannula, and the distal end (404) of the microtube of the tubular structure is in a state of protruding from the distal end of the injection needle or cannula when it is attached. Accordingly, by cutting the protruding distal end of the microtube (including the sealing portion) with scissors or the like and extruding the microtube from the injection needle or cannula, the microtube can be applied to the transplantation site (see Fig. 4).

### 6. Cell-Based Pharmaceutical Product Containing Retinal Pigment Epithelial Cells

As an appropriate example of the cell-based pharmaceutical product of the present invention, a cell-based pharmaceutical product containing retinal pigment epithelial cells is described below.

The retinal pigment epithelial (RPE) cells refer to epithelial cells constituting the retinal pigment epithelium and progenitor cells thereof. The RPE cells may be those isolated from a patient or those derived from stem cells such as pluripotent stem cells. Since the number of RPE cells obtained from a patient is limited, it is necessary to proliferate and expand the RPE cells for transplantation. In the present invention, by culturing and expanding the cells within the microtube composed of a semipermeable membrane, a cell-based pharmaceutical product in which the RPE cells are preloaded in a transplantation device can be obtained.

The method for culturing RPE cells is basically according to WO2022/230977. As the basal medium, a medium commonly used for culturing animal cells may be used. For example, as the basal medium, BME medium, BGJb medium, CMRL 1066 medium, Glasgow MEM (GMEM) medium, Improved MEM Zinc Option medium, IMDM medium, Medium 199, Eagle's MEM medium, αMEM medium, DMEM medium, F-12 medium, DMEM/F12 medium, IMDM/F12 medium, Ham's medium, RPMI 1640 medium, Fischer's medium, or a mixture thereof may be used.

The medium may be a serum-containing medium or a serum-free medium. The serum-free medium may contain a serum substitute. The serum substitute may be a commercially available product, and for example, Knockout^{™} Serum Replacement (KSR), Chemically-defined Lipid concentrated (manufactured by Life Technologies), Glutamax^{™} (manufactured by Life Technologies), B27 (manufactured by Life Technologies), N2 supplement (manufactured by Life Technologies), and ITS supplement (manufactured by Life Technologies) may be used.

The serum-free medium may contain, as appropriate, fatty acids or lipids, amino acids (for example, non-essential amino acids), vitamins, growth factors, cytokines, antioxidants, 2-mercaptoethanol, pyruvic acid, buffering agents, inorganic salts, and the like.

A ROCK inhibitor may be added to the medium. Examples of the ROCK inhibitor include Y-27632 dihydrochloride, Y-27632, Fasudil Hydrochloride, Chroman 1, SLx-2119, HSD1590, GSK269962A hydrochloride, Exoenzyme C3, clostridium botulinum, Ripasudil, Afuresertib, Thiazovivin, GSK269962A, RKI-1447, Y-33075, GSK429286A, AT13148, H-1152 dihydrochloride, Y-33075 dihydrochloride, LX7101, SAR407899, ROCK-IN-2, Afuresertib hydrochloride, Hydroxyfasudil, GSK180736A, BDP5290, SR-3677, CCG-222740, CMPD101, Rho-Kinase-IN-1, SAR407899 hydrochloride, ROCK inhibitor-2, ZINC00881524, H-1152, Hydroxyfasudil hydrochloride, Fasudil, ROCK2-IN-2, Verosudil, SB-772077B dihydrochloride, GSK-25, CRT0066854 hydrochloride, Ripasudil free base, ROCK-IN-1, and the like, with Y-27632 dihydrochloride and Y-27632 being preferred. The concentration of the ROCK inhibitor contained in the medium is usually 0 µM to 20 µM, and preferably 2 µM to 10 µM. The ROCK inhibitor is preferably added when aggregating RPE cells; however, the ROCK inhibitor is not necessarily required in the formulation after culture.

The density of RPE cells to be cultured is not particularly limited, but is usually 2.5 × 10³ cells/mL or more, preferably 2.5 × 10³ cells/mL to 5 × 10⁵ cells/mL, and more preferably 1 × 10⁵ cells/mL to 2 × 10⁵ cells/mL.

The culture time of the RPE cells is not particularly limited, but is usually from 1 day to 30 days, and preferably from 2 days to 7 days.

The culture temperature is, for example, from about 30°C to about 40°C, and preferably about 37°C. The CO₂ concentration is, for example, from about 1% to about 10%, and preferably about 5%.

At least a part of the RPE cells cultured and expanded within the microtube forms aggregates similar to string-like aggregates. By using the transplantation device of the present invention, it is possible to transport RPE cells to a medical institution while maintaining the form of aggregates and to easily load them into the injection needle or cannula for use.

The cell-based pharmaceutical product containing RPE cells can be used for the treatment of diseases based on disorders of the retinal pigment epithelium or for patients having atrophy or damage of the retinal pigment epithelium. Examples of diseases based on disorders of the retinal pigment epithelium or diseases accompanied by atrophy or damage of the retinal pigment epithelium include ophthalmic diseases such as age-related macular degeneration, retinitis pigmentosa, and related diseases such as crystalline retinopathy; retinal pigment epithelial tears, macular dystrophy, cone-rod dystrophy, rod-cone dystrophy, macular hole, degenerative myopia, and traumatic macular diseases.

### [Examples]

The following specifically describes the present invention based on examples, but the present invention is not limited to the examples.

### Test Example 1:

In accordance with a previous report (WO2022/230977), a string-like aggregate of retinal pigment epithelial cells was prepared. The string-like aggregate was seeded in a culture dish and cultured for 2 days.

The culture was carried out in accordance with the description of WO2022/230977 using an RPE maintenance medium containing a ROCK inhibitor (Y-27632). The "RPE maintenance medium" contains DMEM (low glucose) (Sigma-Aldrich), 30% F-12 (Sigma-Aldrich), 2% L-glutamine solution (Sigma-Aldrich), 2% B-27^{TM} supplement (50X) (Thermo Fisher Scientific Inc.), and a gentamicin solution (Sigma-Aldrich).

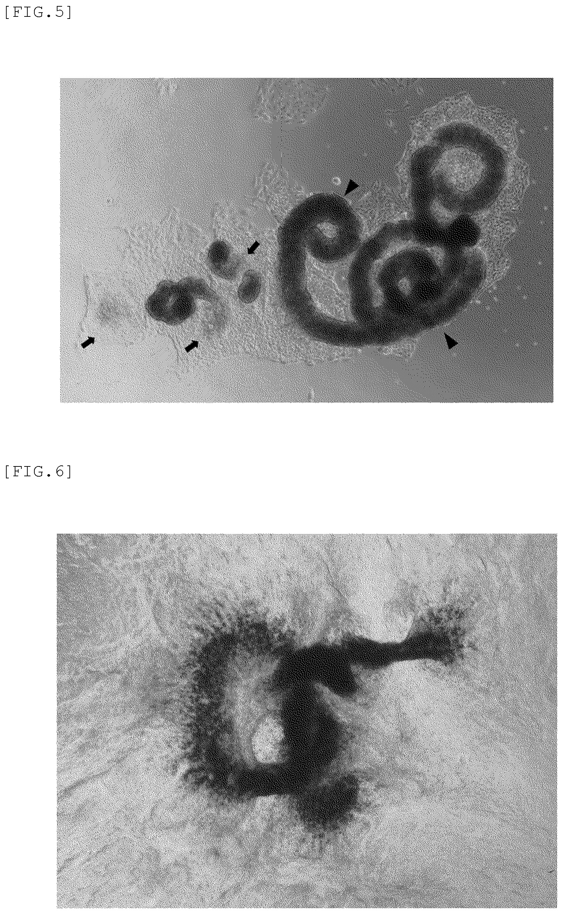

The release of individual retinal pigment epithelial cells from fragmented aggregates (arrows) tended to occur earlier than the release from robust aggregates (arrowheads) (Fig. 5). From these results, it was suggested that string-like aggregates may exhibit high effectiveness as a transplant material even when they are fragmented into smaller pieces to a certain extent.

### Test Example 2:

In accordance with a previous report (WO2022/230977), a string-like aggregate of retinal pigment epithelial cells was prepared, placed in a container filled with an RPE maintenance medium and hermetically sealed, and preserved under anoxic conditions at 25°C for 13 days, after which it was seeded in a culture dish and cultured for 14 days at 37°C under 5% CO₂. From the string-like aggregate that had undergone preservation, retinal pigment epithelial cells migrated in a manner similar to that observed in the string-like aggregate that had not undergone preservation, and during the 14-day culture period, the engraftment area of individual retinal pigment epithelial cells continued to expand (Fig. 6). From these results, it was confirmed that RPE cells can be preserved in a culture medium under anoxic conditions for at least 13 days, and can be maintained and transported in an anoxic and non-drying state.

### Example 1:

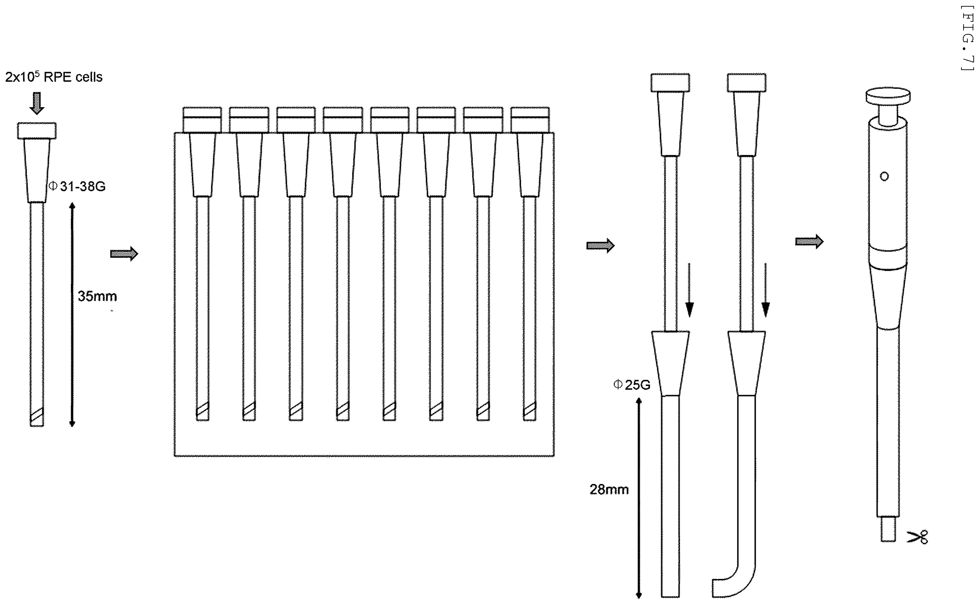

A tubular structure having a configuration (length: 33 millimeters, outer diameter: 31 gauge) similar to that of an inner tube of a cannula (PolyTip Cannula; MedOne#3218, or the like) used for transplantation of string-like aggregates of retinal pigment epithelial (RPE) cells to a subretinal space is prepared using a semipermeable membrane permeable to a cell culture medium, and aggregates are formed by culturing retinal pigment epithelial cells within the lumen thereof (see Fig. 7a). The culture is carried out in accordance with the description of WO2022/230977 using an RPE maintenance medium containing a ROCK inhibitor (Y-27632).

The cultured RPE cells are placed in a container filled with a culture medium together with the tubular structure, and transported while being sealed (see Fig. 7b). As indicated by Test Example 2, the string-like aggregates can be preserved in a culture medium under anoxic conditions for at least 13 days, and are considered to be capable of being transported to and provided at domestic and overseas hospital facilities.

A pharmaceutical product containing RPE cells is used in a manner similarly to a case of a subretinal transplantation cannula with an outer tube (PolyTip Cannula; MedOne#3218, or the like) by inserting the tubular structure into an injection needle (length: 28 millimeters, outer diameter: 25 gauge) corresponding to the outer tube of the subretinal transplantation cannula (PolyTip Cannula; MedOne#3218, or the like) (see Fig. 7c). The outer tube may be selected, according to the operator's preference, from a straight type or a distally bent type.

The above-described procedures of production, transport, and use are briefly summarized below.

### Production:

1) Retinal pigment epithelial cells prepared by a method similar to that described in WO2022/230977 are suspended in an RPE maintenance medium at a cell density of 1 × 10⁸ cells/ml.
2) 2 µl of a cell suspension containing 2 × 10⁵ retinal pigment epithelial cells is injected into a tubular structure composed of a semipermeable membrane having a length of 35 millimeters and an outer diameter of 31 to 38 gauge.
3) An end of the tubular structure at the opposite side of the injection port is closed.
4) The tubular structure is placed in a container filled with the RPE maintenance medium, and cultured at 37°C under 5% CO₂ for 24 to 48 hours.

### Transport:

1) The RPE maintenance medium is filled up to the upper portion of the tubular structure, and the tubular structure is closed with a water-resistant and air-impermeable seal.
2) The closed tubular structure is placed and sealed in a container filled with the RPE maintenance medium, and transported under anoxic conditions.

### Use:

1) The tubular structure transported under anoxic conditions in the container filled with the RPE maintenance medium is unpacked and inserted into a 25G outer tube of an ophthalmic transplantation needle.
2) The seal at the upper portion of the tubular structure is removed, and a composite structure of the transplantation needle (outer tube) and the tubular structure (inner tube) is attached to an ophthalmic transplantation microsyringe.
3) A closed distal end portion of the tubular structure is cut, and the RPE cells (aggregates) are extruded from the tubular structure and transplanted to a subretinal space.

The combination of the inner tube encapsulating cells and the outer tube of the injection needle enables long-term transport of cells that are difficult to handle, such as a string-like aggregate, and reduces procedures immediately before transplantation at the transplantation facility. The above-described method is applicable to not only RPE cells but also to other cells that are difficult to transport or to load into transplantation devices. This makes it possible to provide high-quality cells in a state suitable for transplantation to a greater number of medical facilities.

### Example 2:

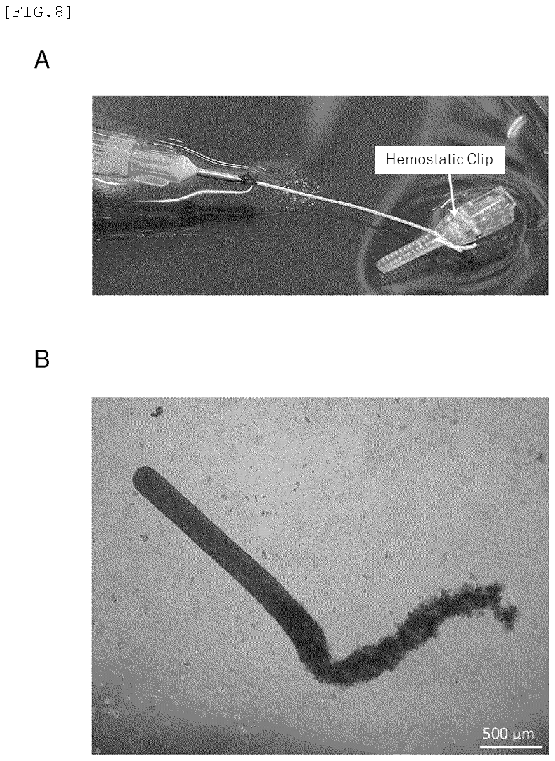

A suspension of cells (human hepatocellular carcinoma-derived cell line HepG2, 5 × 10⁷ cells/mL to 1 × 10⁸ cells/mL) was introduced from the injection portion (104) into the microtube (101) of the inner tube (100). Thereafter, the end portion (105) of the microtube at the opposite side of the injection port was sealed with a hemostatic clip (see Fig. 8A), and the microtube (101) was placed in a reservoir (302) of a culture container (300) filled with a culture medium (303) such that the longitudinal axis of the microtube (101) was aligned with the direction of gravity, and the culture container was set in a culture apparatus to culture the cells. After 24 hours of culture, the hemostatic clip was removed, and the microtube (101, 301) was cut open with scissors at a position approximately 1 mm toward the injection port from the connection point between the clip and the microtube (101). The cell aggregates were then extracted from the end portion (105, 304) of the microtube by feeding a medium through the injection portion (104). The extracted cells had formed an aggregate (Fig. 8B).

All prior art documents cited in the present specification are incorporated herein by reference.

### DESCRIPTION OF REFERENCE SIGNS

- 100:: Inner tube (tubular structure)
- 101:: Microtube composed of semipermeable membrane
- 102:: Transplantation material containing cells
- 103:: Injection port
- 104:: Injection portion
- 105:: Sealing of microtube end portion
- 200:: Outer tube (injection needle or cannula)
- 201:: Needle tube portion
- 202:: Distal end of injection needle or cannula
- 300:: Culture container in which inner tube (tubular structure) is arranged
- 301:: Inner tube (tubular structure)
- 302:: Culture container
- 303:: Reservoir (filled with culture medium)
- 304:: Sealing of microtube end portion
- 305:: Sealing means of injection port (plug, seal, cap)
- 400:: Outer tube (injection needle or cannula) in which inner tube (tubular structure) is set
- 401:: Inner tube (tubular structure)
- 402:: Outer tube (injection needle or cannula)
- 403:: Syringe (external surgical device)
- 404:: Distal end of inner tube (tubular structure)

## Claims

1. A transplantation device comprising:
an inner tube; and
an outer tube, wherein
the inner tube is a tubular structure that includes a microtube that is capable of encapsulating a transplantation material containing cells and composed of a semipermeable membrane and an injection portion provided with an injection port for supplying the cells to the microtube,
the outer tube is an injection needle or cannula that includes a needle tube portion having an inner diameter capable of accommodating the microtube, and
the transplantation device is used by attaching the inner tube to the outer tube.

2. A transplantation device as a tubular structure comprising:
a microtube that is capable of encapsulating a transplantation material containing cells and composed of a semipermeable membrane; and
an injection portion provided with an injection port for supplying the cells to the microtube, wherein
the transplantation device has a structure configured to be used by being attached to an interior of an injection needle or cannula that includes a needle tube portion having an inner diameter capable of accommodating the microtube.

3. The transplantation device according to claim 1 or 2, wherein
the semipermeable membrane is composed of one or a combination of two or more selected from the group consisting of polylactic acid (PLA), polyglycolic acid (PGA), lactic acid-glycolic acid copolymer (PLGA), polyhydroxy acids, polycaprolactone, polycarbonate, polyamide, polyethylene, polyurethane, polyarylate, polysulfone, polyethersulfone, polyester, polystyrene, polyvinyl alcohol, polyvinyl acetate, polyvinyl chloride, polyvinyl fluoride, polyvinyl imidazole, chlorosulfonated polyolefin, polyethylene oxide, polyphosphazene, polyamino acids, polyorthoester, polyacetal, polycyanoacrylate, polytetrafluoroethylene (PTFE), biodegradable polyurethane, polyvinylidene fluoride, polytetrafluoroethylene, cellulose acetate, polyacrylonitrile, polyacrylate, ethylene-vinyl acetate polymer, acyl-substituted cellulose acetate, polymethyl methacrylate, polypropylene, regenerated cellulose, and derivatives thereof.

4. The transplantation device according to claim 1 or 2, wherein
the microtube has an inner diameter of 20 µm to 2000 µm.

5. The transplantation device according to claim 1 or 2, wherein
the semipermeable membrane has a pore diameter of 0.01 µm to 10 µm.

6. The transplantation device according to claim 1 or 2, further comprising one or more of (1) to (3) below:
(1) an end portion of the microtube at an opposite side of the injection port is sealable;
(2) the injection port is sealable; and
(3) the injection needle or cannula includes an attachment portion to an external surgical device.

7. A cell-based pharmaceutical product comprising
a transplantation device preloaded with a transplantation material containing cells, wherein
the transplantation device includes an inner tube and an outer tube,
the inner tube is a tubular structure that includes a microtube that is capable of encapsulating the transplantation material containing cells and composed of a semipermeable membrane and an injection portion provided with an injection port for supplying the cells to the microtube,
the outer tube is an injection needle or cannula that includes a needle tube portion having an inner diameter capable of accommodating the microtube, and
the cell-based pharmaceutical product is used with the inner tube attached to the outer tube.

8. A cell-based pharmaceutical product comprising
a transplantation device preloaded with a transplantation material containing cells, wherein
the transplantation device is a tubular structure including a microtube that is capable of encapsulating a transplantation material containing cells and composed of a semipermeable membrane and an injection portion provided with an injection port for supplying the cells to the microtube, and
the transplantation device has a structure configured to be used by being attached to an interior of an injection needle or cannula that includes a needle tube portion having an inner diameter capable of accommodating the microtube.

9. The cell-based pharmaceutical product according to claim 7 or 8, wherein
the cells are one or two or more selected from stem cells, progenitor cells, somatic cells, and cells induced to differentiate from the stem cells or the progenitor cells.

10. The cell-based pharmaceutical product according to claim 7 or 8, wherein
the cells include retinal pigment epithelial cells.

11. The cell-based pharmaceutical product according to claim 7 or 8, wherein
at least a part of the cells forms an aggregate in the microtube.

12. The cell-based pharmaceutical product according to claim 7 or 8, wherein
the semipermeable membrane is composed of one or a combination of two or more selected from the group consisting of polylactic acid (PLA), polyglycolic acid (PGA), lactic acid-glycolic acid copolymer (PLGA), polyhydroxy acids, polycaprolactone, polycarbonate, polyamide, polyethylene, polyurethane, polyarylate, polysulfone, polyethersulfone, polyester, polystyrene, polyvinyl alcohol, polyvinyl acetate, polyvinyl chloride, polyvinyl fluoride, polyvinyl imidazole, chlorosulfonated polyolefin, polyethylene oxide, polyphosphazene, polyamino acids, polyorthoester, polyacetal, polycyanoacrylate, polytetrafluoroethylene (PTFE), biodegradable polyurethane, polyvinylidene fluoride, polytetrafluoroethylene, cellulose acetate, polyacrylonitrile, polyacrylate, ethylene-vinyl acetate polymer, acyl-substituted cellulose acetate, polymethyl methacrylate, polypropylene, regenerated cellulose, and derivatives thereof.

13. The cell-based pharmaceutical product according to claim 7 or 8, wherein
the microtube has an inner diameter of 20 µm to 2000 µm.

14. A method for producing the cell-based pharmaceutical product according to claim 7 or 8, comprising:
preparing the transplantation device; and
injecting a culture medium containing cells into the microtube of the tubular structure of the transplantation device and culturing the cells by placing the tubular structure in a reservoir of a culture container filled with a culture medium.

15. The method according to claim 14, comprising
sealing, after the culturing, an end portion of the microtube at an opposite side of the injection port.

16. The method according to claim 14, comprising
sealing the injection port of the tubular structure after the culturing, and holding the tubular structure in the reservoir under anoxic conditions.

17. A kit comprising:
the transplantation device according to claim 1 or 2; and
a culture container, wherein
the culture container includes a reservoir configured to be filled with a culture medium and to allow cell culture with the tubular structure placed in the reservoir.

18. The kit according to claim 17, wherein
the culture container is capable of holding the tubular structure in the reservoir under anoxic conditions.
